# EUROPEAN PATENT APPLICATION

(11) **EP 4 467 188 A1**
(43) Date of publication of application: **27.11.2024**
(21) Application number: 23174833.6
(22) Date of filing: 23.05.2023
(51) Int. Cl.: A61N 2/00, A61N 2/02

(54) **MAGNETIC-FIELD APPLICATOR FOR FEET**

(71) Applicant: Iskra Medical d.o.o., 1000 Ljubljana (SI)
(72) Inventor: JELENC, Joze, 4244 Podnar (SI); LILIC, Alen, 6240 Hrpelje-Kozina (SI); KRPAN, Zan, 5000 Nova Gorica (SI); JELENC ,Jure, 4244 Podnart (SI)
(74) Representative: Bardehle Pagenberg Partnerschaft mbB Patentanwälte Rechtsanwälte

(57) **Abstract**

The present invention relates to a magnetic-field applicator comprising a treatment surface adapted to receive at least one foot in an upright orientation and at least one stimulation coil.

## Description

### 1. Field of the Invention

The present invention relates to magnetic stimulation, more specifically to a magnetic-field applicator for feet and a magnetic-stimulation system comprising such magnetic-field applicator. Furthermore, the present invention relates to a method for magnetic stimulation of feet using such magnetic-field applicator.

### 2. Description of the Prior Art

Out of all body parts of an individual, the feet are furthest away from the individual's heart. This is why they are most susceptible to cardiovascular and neurological problems. Moreover, the feet's bone structures and joints experience high forces, often exerted onto them by the individual's entire bodyweight, which may lead to skeletal problems. Multiple feet pathologies arise out of their distal location (with respect to the heart) and/or their exposure to high forces.

Common treatments for cardiovascular, neurological and/or skeletal problems, also in feet, comprise specific drug prescriptions, special diets, the use of medical devices, surgery, etc. Generally, the combination of different approaches and therapies has been shown to yield enhanced results and to in turn further improve the overall health of the respective individual.

At least some cardiovascular, neurological and/or skeletal problems, and/or diseases causing such, may (also) be treated using (high-intensity) magnetic stimulation. Some of the many benefits of magnetic stimulation are increased local blood and/or lymph flow, enhanced muscle tonus and activation of sensory nerves. In addition, magnetic stimulation is non-invasive and easy to use. The effect of high-intensity electromagnetic stimulation on nerves and the motoric response is well described, e.g., in textbooks on transcranial stimulation as well as other sources. In short, rapid changes in the intensity of a magnetic field may induce an electric current in bodily tissue (electromagnetic induction). Once the current reaches a certain threshold, a so-called neuron action potential is achieved. This causes the neuron to activate, which eventually leads to a muscle contraction. Generally suitable parameter ranges, e.g., for the intensity of the magnetic field, needed to achieve this are discussed variously in the prior art.

Some exemplary, common pathologies for which magnetic stimulation specifically of the feet is expected to bring about beneficial results are described in the following.

Due to occupational hazards (e.g., the requirement to stand for extended periods of time) and/or the use of bad (e.g., ill-fitting) footwear feet muscles may grow tense or strained. In more extreme cases plantar fasciitis can occur. Plantar fasciitis involves an inflammation of a thick band of tissue that runs across the bottom of the feet and that connects the heel bone to the toes (plantar fascia). Magnetic stimulation may address such problems and/or ease their symptoms because it has a muscle-relaxing effect (muscle-spasm relaxation) and/or because it increases circulation, thereby soothing the inflammation.

When feet are exposed to constant stress, calcium deposits may build up on the bottom of the heel bone. As a result, a calcaneal spur (also known as a heel spur) may form. A calcaneal spur is a bony outgrowth from the calcaneal tuberosity (heel bone), usually accompanied by a sharp pain in the region surrounding the spur. The increased local circulation caused by magnetic stimulation may reduce calcination and hence inhibit the formation of calcaneal spurs, as well as other types of calcination of the heel.

Tarsal tunnel syndrome (TTS) occurs due to tibial nerve damage. The tibial nerve runs through the tarsal tunnel, a passage of bones and ligaments in the ankle. Symptoms may include pain and/or a burning and/or tingling sensation in the bottom of feet and/or toes. Magnetic stimulation and its muscle-relaxing as well as circulation-enhancing effects may ease the symptoms of TTS.

According to traditional Chinese medicine and foot reflexology, the feet comprise several acupuncture points. These acupuncture points correspond to different body parts and/or organs. Stimulating these acupuncture points is believed to have multiple benefits, including reducing stress, aiding digestion, and promoting sound sleep. And magnetic stimulation may be used for such stimulation.

In a similar vein, magnetic stimulation may be used for tibial nerve stimulation, which is a common urge incontinence treatment. When the tibial nerve is stimulated, impulses travel to the nerve roots in the spine to block abnormal signals from the bladder, preventing bladder spasms. Usually, this treatment is delivered using a slim needle that is placed in the ankle where the tibial nerve is located. However, using magnetic stimulation, one may instead - in a non-invasive manner - stimulate a branch of the tibial nerve that is located near the bottom of the feet (lateral plantar nerve). As such, magnetic stimulation can be used as an alternative, non-invasive, non-pharmacological urge incontinence treatment.

Yet, there are no applicators in the prior art that are specifically adapted for magnetic stimulation of feet, even though it stands to reason that such specifically adapted applicators could greatly enhance the stimulation's therapeutic and/or non-therapeutic effect when applied to feet.

### 3. Summary of the Invention

The present invention seeks to remedy this gap in the prior art.

In a first aspect, the present invention relates to a magnetic-field applicator comprising a treatment surface adapted to receive at least one foot in an upright orientation and at least one stimulation coil. The at least one stimulation coil may be positioned below the treatment surface, but may nevertheless be adapted to generate a magnetic field above the treatment surface. Moreover, the applicator may be adapted to be placed on the ground with the treatment surface pointing substantially upwards.

The inventors have found that it is most meaningful and effective to apply magnetic stimulation to feet when they are in an upright orientation. The upright orientation is the most natural orientation of the feet, resulting in particularly good (or adequate) conditions for magnetic stimulation, in particular with a view to blood flow and/or blood pressure throughout the feet.

Throughout this disclosure, an upright orientation of a foot is generally to be understood as the orientation the foot has when it is positioned on the ground, e.g., when the individual is standing still. For example, a foot may be considered to be in an upright orientation if its longitudinal extension - defined, e.g., by a straight line connecting the heel and the ball of the foot - and/or its footprint - defined, e.g., by a plane in which at least two toes and the ball of the foot lie - is perpendicular to the direction of Earth's gravitational pull. Yet, a foot may also (still) be considered to be in an upright orientation if its longitudinal orientation and/or its footprint is angled with respect to the direction of Earth's gravitational pull, i.e., if its longitudinal extension and/or its footprint encloses an angle of at least 10°, more preferably of at least 20°, even more preferably of at least 30°, even more preferably of at least 45°, even more preferably of at least 60°, even more preferably of at least 75°, even more preferably of at least 80°, even more preferably of at least 85°, most preferably of at least 88° with respect to the direction of Earth's gravitational pull. The same understanding applies, *mutatis mutandis,* to the notion of the treatment surface pointing substantially upwards.

As such, a foot may not only be in an upright position when the individual is standing (still). A foot may also be in an upright position when the individual is sitting (down). In fact, it may even be advantageous if the individual is seated (e.g., sitting or sitting down) when using the magnetic-field applicator according to the present invention. With respect to the feet and their orientation, sitting (down) mimics standing (still). Yet, when the individual is sitting (down), the feet do not experience the high forces exerted by the individual's entire bodyweight as they would when the individual is standing (still). This may provide even further enhanced conditions for magnetic stimulation, especially as it may advantageously affect blood flow and/or blood pressure throughout the feet.

Irrespective, the magnetic-field applicator according to the present invention may need to be sturdy enough such as not to break if, e.g., the weight of the legs of an individual (e.g., when the individual is sitting or sitting down, with at least one foot on the applicator) or even the entire bodyweight of the individual (e.g., when the individual is standing or standing still, with at least one foot on the applicator) is applied to it.

Similarly, the magnetic-field applicator according to the present invention may need to be shaped such that it can be placed, e.g., beneath at least one foot and/or on the ground at all, and in particular such that it can safely receive the at least one foot in an upright direction, e.g., without the risk of slipping out of position, e.g., when weight is applied to it.

The magnetic-field applicator according to the present invention allows to exploit the above advantageous effects that arise when feet are stimulated in an upright orientation, whether the individual is standing (still) or sitting (down). As such, the magnetic-field applicator according to the present invention enables particularly good results in the magnetic stimulation of at least one foot, boosting its therapeutic and/or non-therapeutic effects. The feet's bones, joints, muscles and/or nerves as well as the cardiovascular system of the individual receiving magnetic stimulation as a whole may be exposed to the most suitable magnetic stimulation. Notably, the magnetic-field applicator according to the present invention also allows to achieve these results steadily and reliably, i.e., it offers maximum reproducibility.

Besides, the magnetic-field applicator according to the present invention also allows to apply magnetic stimulation to feet in a particularly comfortable manner. Individuals may generally prefer receiving magnetic stimulation at their feet standing (still) or, even more so, sitting (down), rather than, e.g., lying down. Irrespective, the individual is free to use and/or move his/her hands and/or upper body as (s)he pleases while using the magnetic-field applicator, without negatively affecting the therapeutic and/or non-therapeutic effect of the magnetic stimulation. Also, the magnetic-field applicator according to the present invention may be used with or without clothes, e.g., with or without socks, shoes and/or other footwear.

As a consequence, the magnetic-field applicator according to the present invention is also suitable to apply magnetic stimulation to the feet of an individual with limited mobility that needs to receive magnetic stimulation sitting (down), e.g., an individual that needs to use a wheelchair.

It is noted that common, handheld magnetic-field applicators known from the prior art do not provide any of these advantages as they are not adapted to receive at least one foot in an upright orientation. Neither are they sturdy enough, nor are they shaped such that they can be placed, e.g., beneath at least one foot and/or on the ground, let alone such that they can safely receive the at least one foot, e.g., without the risk of slipping out of position, e.g., when weight is applied to them. To the contrary, their shape and functioning - e.g., a cooling function they comprise and/or require - render them unsuitable to be placed, e.g., beneath at least one foot and/or on the ground such that they could (safely) receive at least one foot in an upright orientation without slipping out of position when weight is applied to them.

In contrast, the magnetic-field applicator according to the present invention may in some examples be specifically adapted to be placed on the ground (with the treatment surface pointing substantially upwards) such that the at least one stimulation coil is held at an elevated position with respect to the ground. This ensures proper (air) cooling of the at least one stimulation coil even when (or even though) the magnetic-field applicator is placed on the ground. Notably, air cooling is generally easier to implement than, e.g., liquid cooling, and also less prone to malfunctions. In some examples, air cooling may be implemented or supported by one or more ventilators arranged in, on and/or near the magnetic-field applicator and/or the at least one stimulation coil. Additionally or alternatively, air - or, more generally, any gas used for cooling - may be provided to the magnetic-field applicator and/or the at least one stimulation coil via one or more tubes, optionally using a pump. The present invention may allow to exploit these advantages of air cooling also in an applicator specifically adapted to apply magnetic stimulation to at least one foot in an upright orientation, which applicator may, e.g., be placed on the ground to this end.

However, in some examples, the at least one stimulation coil may alternatively or additionally be liquid (e.g., water and/or oil) cooled. Liquid cooling may be more effective than air cooling. It may hence be more suitable for certain applications, depending on the parameters used to generate one or more magnetic fields by the at least one stimulation coil (magnetic field intensity, magnetic field change rate, current, pulse duration, etc., discussed in more detail below). Liquid may be provided to the magnetic-field applicator and/or the at least one stimulation coil via one or more tubes, optionally using a pump.

In some examples, when the magnetic-field applicator is placed on the ground, at least a portion of the treatment surface and/or at least a portion of the at least one stimulation coil may enclose a first angle with respect to the ground, preferably of at most 45°, more preferably of at most 15°, most preferably of about 2° to 8°. This allows the individual receiving magnetic stimulation to assume a particularly comfortable position, especially when sitting down, however without significantly affecting the therapeutic and/or non-therapeutic effect of the stimulation. Rather to the contrary, if at least a portion of the treatment surface and/or at least a portion of the at least one stimulation coil is angled with respect to the ground as described, this may nudge the individual to sit (down) while receiving magnetic stimulation at at least one foot. And as discussed above, sitting (down) may provide even further enhanced conditions for magnetic stimulation, e.g., because it may advantageously affect blood flow and/or blood pressure throughout the feet. That is, the magnetic-field applicator according to the present invention allows the individual receiving magnetic stimulation to assume the most suitable and appropriate, yet comfortable position for receiving magnetic stimulation at at least one of his/her feet, because the magnetic-field applicator according to the present invention may be adapted/adjusted such that it takes into account the most natural orientation and/or position of an individual and/or his/her feet.

In some examples, said first angle may be adjustable. For example, it may be adjusted using one or more, preferably adjustable, spacers. This allows to take an individual's preferences into account. For example, some individuals may prefer to receive magnetic stimulation at at least one foot standing (still), such that they require the first angle to be rather small. Yet, other individuals may prefer to receive magnetic stimulation at at least one foot sitting (down), though not upright but rather reclined, such that they require the first angle to be rather large. Sometimes, it may also be necessary to adjust the first angle to take into account an individual's anatomy. For example, individuals with longer legs may need to place the magnetic-field applicator further away from, e.g., the chair they are sitting on to properly place at least one foot thereon. This in turn requires the first angle to be larger. Yet, individuals with shorter legs may need to place the magnetic-field applicator closer to, e.g., the chair they are sitting on to properly place at least one foot thereon. This in turn requires the first angle to be smaller. At any rate, if the first angle is adjustable, magnetic stimulation of the at least one foot may be more comfortable for the individual receiving it, but also more effective.

In some examples, the treatment surface and/or the at least one stimulation coil may be movable relative to each other, e.g., manually or using one or more motors as will be described in more detail below. Such relative movement may be effectuated before or during an ongoing magnetic stimulation. It may take into account the size and/or shape of the at least one stimulation coil and the size and/or shape of the at least one foot to be received by the treatment surface. Additionally or alternatively, it may take into account feedback provided by the individual receiving magnetic stimulation. In some examples, the treatment surface may be stationary and the at least one stimulation coil may be moved with respect thereto. For example, the at least one stimulation coil may be movable within the magnetic-field applicator, along at least one, preferably two, most preferably three dimensions. Additionally or alternatively, it may also be rotatable about at least one, preferably two, most preferably three dimensions or axes, resepctively. In some examples, the at least one stimulation coil may be stationary and the treatment surface may be moved with respect thereto. In some examples, neither the treatment surface nor the at least one stimulation coil is stationary, but both may be moved. The magnetic field(s) generated by the at least one stimulation coil generally do(es) not only vary in time but also in space. Thus, by moving the treatment surface - and with it the at least one foot placed thereon -relative to the at least one stimulation coil, and/or *vice versa,* it may be influenced how and/or where the magnetic field(s) stimulate(s) the at least one foot. For example, the magnetic field(s) may generally be strongest at a specific point with respect to the at least one stimulation coil, and by moving the treatment surface and/or the at least one stimulation coil relative to each other, this specific point may be placed in different locations throughout the at least one foot placed on the treatment surface. More generally, by moving the treatment surface and/or the at least one stimulation coil relative to each other, the stimulation sensation and/or intensity felt at the at least one foot may be altered. As a result, the stimulation may be rendered as comfortable as possible for the individual receiving it, but also more effective, enhancing therapeutic and/or non-therapeutic results.

Preferably, one or more motors and/or one or more springs may be used to move the treatment surface and/or the at least one stimulation coil relative to each other. This may allow to perform a series of relative movements precisely and, if needed, rapidly. This may in turn be exploited to (successively) alter the stimulation sensation and/or intensity felt at the at least one foot, e.g., in a predefined manner. For example, this may be used to create a sensation that a stimulus is wandering through the at least one foot. Similarly, considering that magnetic stimulation may cause muscle contractions, this may be used to create the sensation of a massage of the at least one foot. The one or more motors may comprise a position log. This may also allow to adjust (e.g., move and/or orient) the at least one stimulation coil and the treatment surface relative to each other automatically, e.g., according to a pre-programmed position determined based on the size and/or shape of the at least one stimulation coil, the size and/or shape of the at least one foot to be received by the treatment surface, and/or the desired distribution of the magnetic field(s) to be generated within the at least one foot and/or the body of the individual receiving magnetic stimulation more generally. This may further increase comfort and effect of the magnetic stimulation of at least one foot.

In some examples, a magnetic core is placed inside and/or around the at least one stimulation coil. A magnetic core may be a piece of magnetic material with a high magnetic permeability. A magnetic core may be used to confine and guide magnetic fields. This may in turn allow to further modify the properties of the magnetic field(s) the at least one stimulation coil generates, such as to further adapt it/them for the magnetic stimulation of at least one foot. The magnetic core may optionally be movable relative to the treatment surface and/or the at least one stimulation coil. Similar effects as described above may be obtained by moving the magnetic core instead of, or in addition to, the at least one stimulation coil and/or the treatment surface relative to the respective other component(s). Preferably, one or more motors and/or one or more springs may be used to move the magnetic core relative to the treatment surface and/or the at least one stimulation coil, similar as was described above already for the case that the treatment surface and/or the at least one stimulation coil may be movable relative to each other.

In some examples, the at least one stimulation coil may be sized and/or shaped to correspond to the at least one foot. Naturally, the properties (e.g., size, density, shape and depth) of the magnetic field(s) the at least one stimulation coil generates depend on the size and/or shape of the at least one stimulation coil. For example, the size and/or shape of the at least one stimulation coil may determine the spatial distribution of the, e.g., alternating, magnetic field(s) and how and/or where they are applied to the feet and/or legs of an individual receiving magnetic stimulation using the magnetic-field applicator. By choosing the size and/or shape of the at least one stimulation coil to correspond to the at least one foot, it may be ensured that the magnetic field(s) generated by the at least one stimulation coil comprise(s) properties that are suitable, or even optimal, for treating the at least one foot, e.g., in terms of size, density, shape and depth of the magnetic field(s). The result is an enhanced therapeutic and/or non-therapeutic effect of the stimulation. Magnetic-field applicators known from the prior art lack such stimulation coils that are specifically sized and/or shaped to effectively, let alone optimally, stimulate at least one foot.

Suitable shapes for the at least one stimulation coil may comprise a figure-of-eight shape or an oval shape. Both, but especially the latter, are well-suited for applying magnetic stimulation to at least one foot considering that feet typically comprise a generally elongate outline that may be said to resemble an oval and/or a figure-of-eight shape. That is, such stimulation coils may generally be said to mimic the shape of feet.

Suitably, the at least one stimulation coil may comprise a diameter of 15 cm to 40 cm, preferably of 20 cm to 35 cm. As such, the at least one stimulation coil may correspond well in size to the average foot of the worldwide population. But also other sizes may be used for individual with smaller or larger feet.

In some examples, the treatment surface may be adapted to receive two feet in an upright orientation. This may allow to apply magnetic stimulation to both feet of an individual at once. This may in turn render the respective treatment more (time-)effective and it may also reduce the risk that the two feet of an individual are, inadvertently and/or unintentionally, treated differently. Also, if the treatment surface is adapted to receive two feet in an upright orientation, enhanced stimulation sensations may be created. For example, a sensation may be created that a stimulus is wandering from one foot to the other, as it was described above already with respect to a sensation of a stimulus wandering through (at least) one foot.

In some examples, the at least one stimulation coil may comprise a first stimulation coil and a second stimulation coil placed side-by-side with respect to the treatment surface. Using two coils side-by-side may be advantageous in that it allows to further modify and/or tailor the properties, especially the size and/or shape, of the magnetic field(s) that are to be applied using the magnetic-field applicator. This may be particularly advantageous where the treatment surface is adapted to receive two feet in an upright orientation. Then, the first and second stimulation coils may each correspond to one of the two feet that are to be received by the treatment surface, which may make it easier to size and/or shape the magnetic field(s) to efficiently treat both feet. What is more, using two coils side-by-side may then also allow the two feet of an individual to be treated differently, but nevertheless simultaneously. For example, this may be advantageous where the two feet of an individual suffer from different diseases, or from the same disease, but to a different degree.

Generally, the first stimulation coil comprises a first orientation and the second stimulation coil comprises a second orientation. For stimulation coils comprising a generally elongate shape, their orientation may be determined by their longest/largest dimension/diameter. For example, for a stimulation coil comprising an oval shape (e.g., an oval cross-section), its orientation may be determined by the major axis of the oval shape.

Preferably, the first stimulation coil and the second stimulation coil may be arranged such that the first orientation and the second orientation enclose a second angle of 5° to 40°, most preferably of 25° to 35°. This enables the individual receiving magnetic stimulation to assume a particularly comfortable position, especially when sitting (down), while providing for optimal stimulation results and therapeutic and/or non-therapeutic effect. In their most natural orientation, especially when the individual is sitting (down), the two feet of an individual (or their respective longitudinal extensions) enclose an angle of 5° to 40°, most preferably of 25° to 35°, e.g., 30°. Thus, if the first stimulation coil and the second stimulation coil are arranged at a corresponding angle (and, optionally, sized appropriately), the individual will virtually automatically place his/her feet optimally on the magnetic-field applicator, i.e., such that each foot is placed above, and also aligned with, one of the first stimulation coil and the second stimulation coil, respectively. That is, the magnetic-field applicator according to the present invention allows the individual receiving magnetic stimulation to assume the most suitable and appropriate, yet comfortable position for receiving magnetic stimulation at at least one of his/her feet, because the magnetic-field applicator according to the present invention may be adapted/adjusted such that it takes into account the most natural orientation and/or position of an individual and/or his/her feet.

Optionally, the second angle may also be adjustable. On the one hand, this may be beneficial because it allows to take the individual's preferences and anatomy into account as already described above with respect to the first angle optionally being adjustable. On the other hand, this may be exploited to create different stimulation sensations and/or intensities, perhaps also during an ongoing magnetic-stimulation session. As described elsewhere herein, the position of the treatment surface, with the two feet placed thereon, relative to the at least one stimulation coil - here: the first stimulation coil and the second stimulation coil - crucially impacts the stimulation sensation experienced by the individual and his/her feet. To this end, it may be advantageous if the second angle may be adjusted using one or more motors. Either way, an enhanced stimulation experience may be achieved this way.

In some examples, the treatment surface may comprise at least one bulge adapted to receive at least a part of the at least one foot. For example, the at least one bulge may be adapted to receive a medial part of a forefoot region of the at least one foot, and may be shaped accordingly. Yet, the at least one bulge may also be adapted to receive another part of the at least one foot. Optionally, the shape of the at least one bulge may be adjustable such that it can be adapted to receive different parts of the at least one foot. There may also be multiple bulges, each adapted to receive a different part of the at least one foot. Where the treatment surface is adapted to receive two feet, there may also be a set of bulges adapted to receive mutually corresponding parts of the two feet. To this end, the set of bulges may be arranged (mirror) symmetrically on the treatment surface. Magnetic stimulation using the applicator according to the present invention may cause muscle contractions in the at least one foot, which may in turn urge the at least one foot towards the treatment surface, e.g., in a curling motion. If there is at least one bulge adapted to receive at least a part of the at least one foot, the at least one foot may be urged against the at least one bulge. This may lead to a stretching of muscle(s) and fascia(e) in the at least one foot, e.g., across or along the at least one bulge, especially where the individual uses the magnetic-stimulation applicator, e.g., barefoot or only wearing socks. In turn, the stimulation's therapeutic and/or non-therapeutic effect may be enhanced and the comfort of the individual receiving the stimulation may be increased. These advantages may be further supported if the shape of the at least one bulge is adjustable. Generally, this may allow to adjust the intensity of the stretching. Besides, when the shape of the at least one bulge is adjusted during ongoing magnetic stimulation, this may provide, or pronounce, a sensation of a foot massage. Especially to this end, it may be advantageous if the shape of the bulge can be adjusted using one or more motors.

More generally, the treatment surface may comprise a shape, e.g., protrusions, bulges, recesses and/or indentations, that allow to realize a massaging function. Suitably, the shape of such, e.g., protrusions, bulges, recesses and/or indentations, may be adjusted during magnetic stimulation, e.g., using one or more motors. This may enhance the massage experience. Additionally or alternatively, further elements and/or devices adapted to provide a massaging function may be placed, applied or mounted onto the treatment surface, in particular if the treatment surface is substantially flat.

Additionally or alternatively, the treatment area may comprise a shape, e.g., protrusions, bulges, recesses and/or indentations, that is specifically arranged to accommodate the at least one foot. That is, generally, the treatment surface may not be flat. Rather, it may be adapted to correspond to (e.g., follow or mirror) the anatomy of a human foot, at least partially. Then, it may be said that the treatment surface comprises a shape that forms a footbed for the at least one foot. In some examples, the treatment surface comprises an elevated portion that is adapted to receive at least a part of the at least on foot, e.g., a forefoot part of the at least one foot, or only a medial part of a forefoot part of the at least one foot. All in all, this may render the magnetic-field applicator more comfortable to use, especially when it is used barefoot or only wearing socks. Besides, if the treatment surface is shaped as described, this may help correct placement of the at least one foot (relative to the at least one stimulation coil), in turn supporting proper use of the applicator and hence enhancing stimulation results.

In some examples, the shape of the treatment surface may be adjustable. In some examples, the shape of the treatment surface may be adjustable by placing one or more suitably shaped elements thereon, similar to shoe inserts. This allows to take an individual's preferences and/or anatomy into account, as described elsewhere herein with reference to different aspects of the present invention.

In some examples, the treatment surface may comprise a biocompatible material. For example, the treatment surface may comprise no sharp, hot, cold or in any other way hazardous characteristic. This may be advantageous if the magnetic-field applicator is meant to be used barefoot. The biocompatible material avoids any irritation or even injury of, e.g., the skin of the foot by the treatment surface. Also, it may be easily cleaned and/or disinfected, using regular equipment, before and/or after use, especially if the treatment surface is substantially flat.

Notably, especially when the treatment surface comprises a biocompatible material, but also more generally, the magnetic-field applicator according to the present invention is also suitable for use by individuals that cannot wear footwear of any kind, e.g., due to a disease (diabetes, gout, etc.) or an injury, and/or because they need to wear an immobilization garment (plaster immobilization garment, cast, etc.) at the respective foot.

That said, the magnetic-field applicator according to the present invention is not limited to barefoot use. To the contrary, adjusting the properties (e.g., size, density, shape and depth) of the magnetic field(s) the at least one stimulation coil generates as described, e.g., above, may allow the applicator to be used, e.g., wearing shoes, or any other type of footwear.

The at least one stimulation coil may be adapted to generate a magnetic field at the treatment surface with an intensity of 0.1 to 5 T, preferably with an intensity of 0.5 to 3 T, alternatively with an intensity of 0.1 to 1 T, and/or with a change rate of 500 to 500,000 T/s, preferably with a change rate of 10,000 to 200,000 T/s, and/or upon receiving a current of 500 to 10,000 A, preferably upon receiving a current of 3,000 A to 5,000 A. Additionally or alternatively, the at least one stimulation coil may be adapted to generate a magnetic field at the treatment surface for a duration of 10 to 900 µs, preferably for a duration of 100 to 500 µs. That is, the present invention generally relates to high-intensity magnetic stimulation. Insofar, it distinguishes over prior art that relates to low-intensity magnetic stimulation.

Suitably, the at least one stimulation coil may comprise an inductance of 0.1 to 10,000 µH, preferably of 1 to 500 µH. Stimulation coils with these properties are suitable for use in high-intensity magnetic stimulation; and said diameters correspond well to the size of a human foot, which may enhance stimulation results as described elsewhere herein.

As will be appreciated from the foregoing, the magnetic-field applicator according to the present invention generally allows to be adjusted in many ways, e.g., to the preferences and/or anatomy of the individual receiving magnetic stimulation. This makes it particularly versatile and comfortable to use. Generally, it offers maximum control of parameters.

It is also to be understood that any of the possible adjustments may be made - whether manually and/or using one or more motors and/or one or more springs, whether before, during and/or after magnetic stimulation is applied to the at least one foot - in a predetermined manner, e.g., according to a (pre-determined) configuration that may take into account the size and/or shape of the at least one stimulation coil, the size and/or shape of the at least one foot to be received by the treatment surface, and/or the desired distribution of the magnetic field(s) to be generated within the at least one foot and/or the body of the individual receiving magnetic stimulation more generally. Additionally or alternatively, any of the possible adjustments may be made according to feedback provided by the individual receiving magnetic stimulation.

That said, the parameter ranges mentioned above, especially those for the first angle and for the second angle, were determined based on extensive testing and the suggestions and feedback of experienced medical personnel, such as to maximize therapeutic and/or non-therapeutic effects but also comfort for the individual receiving magnetic stimulation.

In a second aspect, the present invention relates to a magnetic-stimulation system comprising at least one pulse generator and a magnetic-field applicator as described above, wherein the applicator and/or the at least one stimulation coil of the applicator is connectable, e.g., via one or more wires, to the at least one pulse generator. Such system may be used to provide magnetic stimulation using the magnetic-field applicator according to the present invention and to exploit its advantages as described above. Such system, and in particular its at least one pulse generator, may be adapted to generate different modulations, frequencies, time phases and/or intensities for the (pulsed and/or alternating) magnetic field(s) that are generated by the system. If there are two or more stimulation coils in the system, they may be controlled to generate the same or different magnetic field(s), e.g., in terms of intensity. For example, they may generate magnetic field(s) simultaneously or alternatingly. If there are multiple stimulation coils, they may generally also be controlled individually.

In a third aspect, the present invention relates to a method for magnetic stimulation of at least one foot of an individual. The method comprises placing, in an upright orientation, the at least one foot on a treatment surface of a magnetic-field applicator, preferably on the treatment surface of a magnetic-field applicator as described above, and applying one or more pulses by the applicator (e.g., to the at least one foot). Preferably, the one or more pulses may be applied by the applicator while the individual is sitting (down), preferably on a chair. Of course, the individual may also (already) be sitting (down) when placing the at least one foot on the treatment surface in an upright orientation. Alternatively, the one or more pulses may be applied by the applicator while the individual is standing (still). Likewise, the individual may also (already) be standing (still) when placing the at least one foot on the treatment surface in an upright orientation. By using the magnetic-field applicator according to the present invention, such method for magnetic stimulation of at least one foot exploits the advantages described above and achieves particularly good stimulation results and an enhanced therapeutic and/or non-therapeutic effect.

Accordingly, in a fourth aspect, the present invention relates to the use of a magnetic-field applicator as described above in an urge incontinence treatment. As described elsewhere herein, magnetic stimulation may be used for tibial nerve stimulation, which is a common urge incontinence treatment. Specifically, using magnetic stimulation, one may - in a non-invasive manner - stimulate a branch of the tibial nerve that is located near the bottom of the feet (lateral plantar nerve). And the magnetic-field applicator according to the present invention is particularly suitable for stimulating this branch of the tibial nerve. Its use may hence achieve particularly good stimulation results and an enhanced therapeutic and/or non-therapeutic effect of such urge incontinence treatment.

Of course, the magnetic-field applicator according to the present invention may come, or be used, in combination with one or more other (types of) applicators, e.g., in a magnetic-stimulation system as described above and/or in a method for magnetic stimulation as described above. In particular, the magnetic-field applicator according to the present invention may be combined with an applicator adapted to stimulate the pelvic or pelvic floor region of an individual, which may come in the shape of a chair (e.g., with one or more stimulation coils arranged within or under the seat of the chair). As described above, the magnetic-field applicator according to the present invention is well suited to be used sitting (down), such that it may be easily combined with such other applicators comprising the shape of a chair. In fact, the magnetic-field applicator according to the present invention may even be integrated into, or at least be adapted to be attached to, such an applicator comprising the shape of a chair. Combining the magnetic-field applicator according to the present invention with such an applicator comprising the shape of a chair may enable particularly effective magnetic stimulation and may provide a particularly effective urge incontinence treatment.

Additionally or alternatively, the magnetic-field applicator according to the present invention may come, or be used, in combination with one or more other (types of) applicators that are used to, or adapted to, stimulate a leg of the individual, preferably below the knee of the leg. For example, the magnetic-field applicator according to the present invention may be used in combination with a (common) hand-held applicator that is then used to apply one or more additional pulses to the leg of the individual, preferably below the knee of the leg. The one or more other (types of) applicators may be held in place by a stand (e.g., a tripod) and/or arm. Additionally or alternatively, the one or more other (types of) applicators may be strapped to the leg of the individual. The one or more other (types of) applicators may also be integrated into, or at least be adapted to be attached to, the magnetic-field applicator according to the present invention. Depending on where exactly the one or more other (types of) applicators are placed, they may, in combination with the magnetic-field applicator according to the present invention, be used to achieve different therapeutic as well as non-therapeutic effects. For example, the one or more other (types of) applicators may enhance an urge incontinence treatment as enabled by the magnetic-field applicator according to the present invention as described above. The one or more other (types of) applicators may also cure or ameliorate Achilles tendon problems (e.g., injuries or tendinitis) and/or problems in the upper foot.

### 4. Brief Description of the Figures

Possible examples of the present invention will be described below with reference to the following figures:
- Figs. 1A-C:: Different views of an example of a magnetic-field applicator according to the present invention;
- Fig. 2:: Example of a stimulation coil comprising an oval shape according to the present invention;
- Figs. 3A-D:: Examples of uses of a magnetic-field applicator according to the present invention in combination with a hand-held applicator.

### 5. Detailed Description of Possible Examples

For the sake of brevity only a few examples will be described in the following. The skilled person will recognize that the specific features described with reference to these examples may be modified and combined differently and that individual features may also be omitted if they are not essential. The general explanations in the sections above will also be valid for the following explanations.

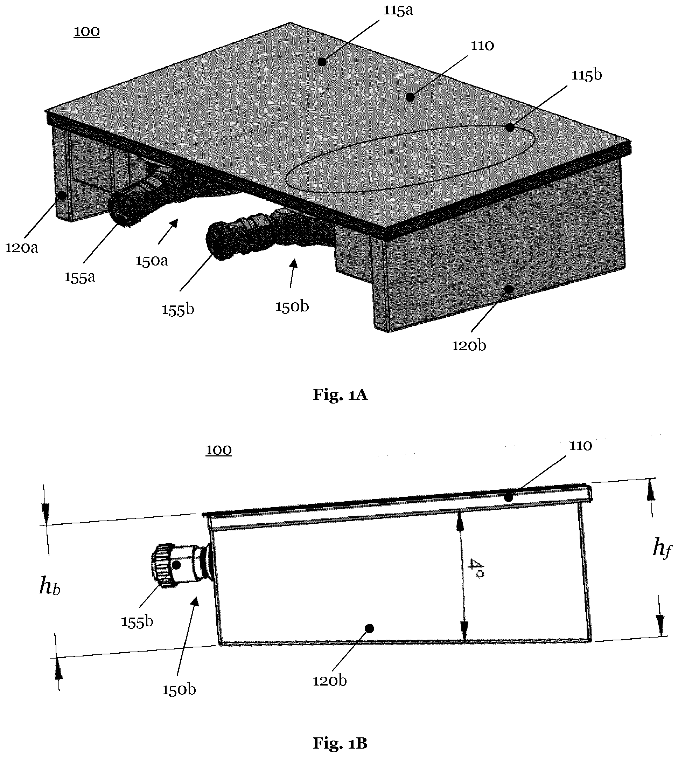

Figs. 1A-C show an example of a magnetic-field applicator 100 according to the present invention. Applicator 100 comprises a treatment surface 110 adapted to receive at least one foot, i.e., two feet, in an upright orientation and at least one stimulation coil, i.e., first stimulation coil 150a and second stimulation coil 150b. The unique shape of applicator 100 is designed to allow the individual using it to assume the most suitable and appropriate, yet comfortable position for magnetic stimulation.

Applicator 100 is adapted to be placed on the ground with treatment surface 110 pointing substantially upwards, as shown in Figs. 1A and 1B. As such, one or more feet placed thereon will inevitable be received in (e.g., assume) an upright orientation.

Specifically, applicator 100 comprises side walls 120a and 120b that support treatment surface 110 when applicator 100 is placed on the ground. That is, when applicator 100 is placed on the ground, one (i.e., the lower) edge each of side walls 120a and 120b is placed on the ground. The respective opposite (i.e., the upper) edges of side walls 120a and 120b abut treatment surface 110.

Sidewalls 120a and 120b as well as treatment surface 110 may comprise, or consist of, one or more strong, sturdy materials. Sidewalls 120a and 120b as well as treatment surface 110, and as such applicator 100 as a whole, are adapted to withstand high mechanical forces. In particular, sidewalls 120a and 120b as well as treatment surface 110, and as such applicator 100 as a whole, are adapted to withstand the full bodyweight of an individual seeking to use applicator 100. That is, applicator 100, e.g., sidewalls 120a and 120b as well as treatment surface 110, will not break if the individual stands (still) on applicator 100, specifically on treatment surface 110. Even more so, applicator 100, e.g., sidewalls 120a and 120b as well as treatment surface 110, will not break if the individual places one or more feet on applicator 100, specifically on treatment surface 110, while sitting (down).

Sidewalls 120a and 120b are mirror symmetric and comprise a trapezoidal shape. In particular, the lower edge and the upper edge of each side wall 120a and 120b are not parallel but angled with respect to each other. As a result, treatment surface 110-which is substantially flat in applicator 100, but may be shaped differently in other examples, as described elsewhere - encloses a first angle with respect to the ground when applicator 100 is placed on the ground. Put differently, applicator comprises a larger height *h_{f}* in its front, and a smaller height *h_{b}* in its back. In the specific case of applicator 100, the first angle is 4°, as indicated in Fig. 1B. Yet, in other examples, the first angle may be different; it may have any value, preferably at most 45°, more preferably at most 15°, most preferably between 2° to 8°. In other examples, the first angle may be adjustable, optionally also using one or more motors.

As mentioned above, treatment surface 110 of applicator 100 is substantially flat, as shown in Figs. 1A and 1B. In other examples, it may however not be substantially flat. For example, treatment surface 110 may instead comprise at least one bulge adapted to receive at least a part of the at least one foot, as described above. Additionally or alternatively, treatment surface 110 may comprise a shape that forms a footbed for the at least one foot it is adapted to receive. For example, if treatment surface 110 is adapted to receive two feet, as it is in applicator 100, it may comprise a shape that forms two footbeds, one for each of the two feet treatment surface 100 is adapted to receive. More generally, treatment surface 110 may be adapted to correspond to (e.g., follow or mirror) the anatomy of a human foot, at least partially. Of course, if treatment surface 100 is adapted to receive two feet, as it is in applicator 100, it may be adapted to correspond to (e.g., follow or mirror) the anatomy of two feet, at least partially. For example, treatment surface 110 may comprise a (non-flat) shape that is mirror symmetric, corresponding to the anatomies of two mirror-symmetric feet of an individual.

Treatment surface 110 may comprise a biocompatible material. This may be advantageous for barefoot use of applicator 100. However, applicator 100 may also be used wearing shoes or any other kind of footwear.

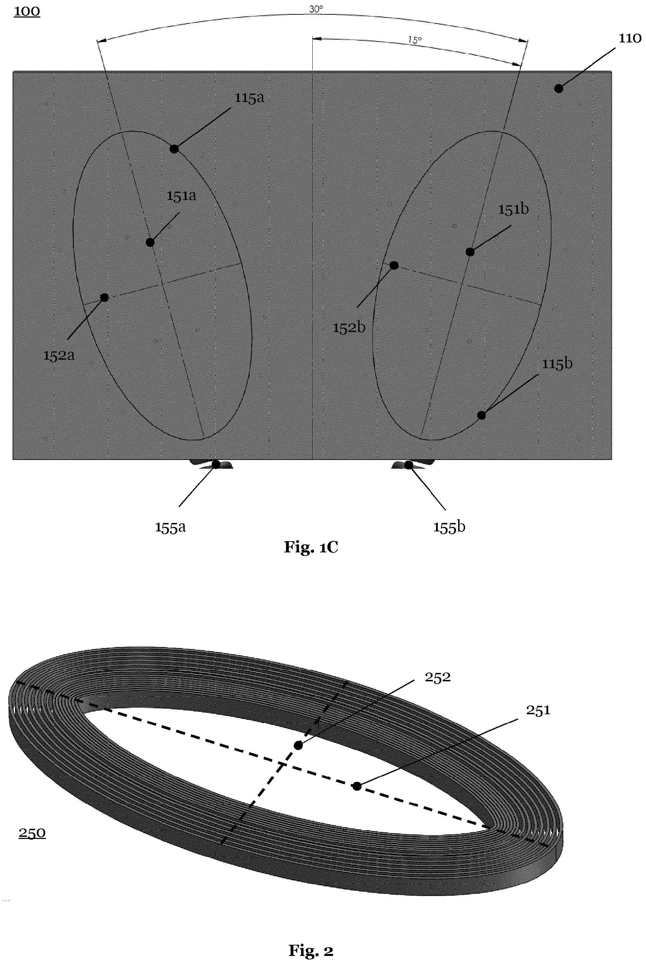

Below treatment surface 110 of applicator 100, first stimulation coil 150a and second stimulation coil 150b are positioned, side-by-side (with respect to treatment surface 110). First stimulation coil 150a and second stimulation coil 150b comprise an identical, flat, oval shape (which will be discussed in more detail below, also with reference to Fig. 2). In applicator 100, the general shape (and size) as well as position of first stimulation coil 150a and second stimulation coil 150b is indicated on treatment surface 110 by respective markings 115a and 115b. Markings 115a and 115b are visible to an individual seeking to use applicator 100, also and especially when applicator 100 is placed on the ground. Markings 115a and 115b may help the individual to properly place his/her feet on applicator 100, e.g., such that the left foot is placed on or within marking 115a and thus centrally over first stimulation coil 150a, and the right foot is placed on or within marking 115b and thus centrally over second stimulation coil 150b.

Flat, oval stimulation coils 150a and 150b are attached, from below, to treatment surface 110. As a result, they both enclose the same first angle with respect to the ground as treatment surface 110. Moreover, stimulation coils 150a and 150b are hence held at an elevated position with respect to the ground. This allows for sufficient cooling, e.g., air cooling, when applicator 100 is in use. When generating one or more magnetic fields, stimulation coils 150a and 150b may heat up considerably, which may render cooling indispensable. Air cooling, as exemplified by applicator 100, may generally be easier to implement than, e.g., liquid cooling, and also less prone to malfunctions. However, as described elsewhere herein, for some applications, liquid cooling may be preferable.

In other examples, stimulation coils 150a and 150b may not both be attached to treatment surface 110. Rather, at least one of, or even both, stimulation coils 150a and 150b may not be attached to treatment surface 100. This may allow for stimulation coils 150a and 150b to be moved relative to treatment surface 110 and hence relative to at least one foot that treatment surface 110 is adapted to receive. In such examples, stimulation coils 150a and 150b may also be moved relative to the remainder of applicator 100, e.g., side walls 120a and 120b. Such movement may be effectuated using one or more motors, possibly for each of stimulation coils 150a and 150b that is movable.

In yet other examples, treatment surface 100, and with it at least one foot it is adapted to receive, may be moved relative to simulation coils 150a and 150b. In such examples, treatment surface 110 may also be moved relative to the remainder of applicator 100, e.g., side walls 120a and 120b. Such movement may be effectuated using one or more motors, the one or more motors acting on treatment surface 110 rather than stimulation coils 150a and 150b as described before for other examples.

As mentioned above, first stimulation coil 150a and second stimulation coil 150b comprise an identical, flat, oval shape. Moreover, stimulation coils 150a and 150b comprise a diameter of 20 cm to 35 cm. The diameter of flat, oval stimulation coils 150a and 150b is determined by major axes 151a and 151b as indicated in Fig. 1C. Accordingly, the width of stimulation coils 150a and 150b, determined by minor axes 152a and 152b as indicated in Fig. 1C, is smaller than 35 cm. As such, stimulation coils 150a and 150b are each sized and shaped to correspond to one of the two feet treatment surface 110 of applicator 100 is adapted to receive.

In other examples, a different number of, or differently shaped, stimulation coils may be employed. For example, a single, larger oval or circular stimulation coil may be used that is large enough to apply magnetic stimulation to both feet that are to be received on treatment surface 110. In other examples, one or more figure-of-eight coils may be used to focus the magnetic stimulation to one or more specific points and/or parts of the at least one foot received by treatment surface 110.

Stimulation coils 150a and 150b may comprise an inductance of 0.1 to 10,000 µH, preferably of 1 to 500 µH. In some examples, a magnetic core may be placed inside and/or around at least one of, or even both, stimulations coils 150a and 150b, also to focus the magnetic stimulation to one or more specific points and/or parts of the feet received by treatment surface 110.

Generally, first stimulation coil 150a comprises a first orientation determined by major axis 151a, and second stimulation coil 150b comprises a second orientation determined by major axis 151b. In applicator 100, first stimulation coil 150a and second stimulation coil 150b are angled with respect to each other. That is, the first orientation, determined by major axis 151a, and the second orientation, determined by major axis 151b, enclose a second angle. In applicator 100, the second angle is 30°, as indicated in Fig. 1C. Yet, in other examples, the second angle may be different; it may have any value, preferably between 5° and 40°, most preferably between 25° and 35°. In other examples, the second angle may be adjustable, optionally using one or more motors.

First stimulation coil 150a and second stimulation coil 150b are arranged symmetrically, side-by-side, below - but also with respect to - treatment surface 110. That is, stimulation coils 150a and 150b are arranged symmetrically with respect to mirror-symmetry axis 111 of treatment surface 110, as indicated in Fig. 1C. As a corollary, the first orientation of first stimulation coil 150a and the second orientation of second stimulation coil 150b both enclose an angle of 15° with respect to mirror-symmetry axis 111, as indicated in Fig. 1C.

First stimulation coil 150a comprises a first connector 155a and second stimulation coil 150b comprises a second connector 155b. Via connectors 155a and 155b, stimulation coils 150a and 150b (and/or applicator 100, respectively) may be connected to at least one pulse generator (not shown in Figs. 1A-C), e.g., both to the same pulse generator, or to two separate pulse generators, to form a magnetic-stimulation system according to the present invention.

Stimulation coils 150a and 150b may (each) receive a current of 500 to 10,000 A, preferably a current of 3,000 A to 5,000 A, from the one or more pulse generators. The stimulation coils 150a and 150b may generate one or more magnetic fields at treatment surface 110 with an intensity of 0.1 to 5 T, preferably with an intensity of 0.5 to 3 T, and/or with a change rate of 500 to 500,000 T/s, preferably with a change rate of 10,000 to 200,000 T/s. The stimulation coils 150a and 150b may generate such one or more magnetic fields for a duration of 10 to 900 µs, preferably for a duration of 100 to 500 µs. That is, stimulation coils 150a and 150b (and the one or more pulse generators they are connected to) are adapted to provide high-intensity magnetic stimulation.

Fig. 2 shows an example of a stimulation coil 250 comprising an oval shape according to the present invention. Similar to stimulation coils 150a and 150b of applicator 100 of Figs. 1A-C, stimulation coil 250 comprises a flat, oval shape. Moreover, stimulation coil 250 comprises a diameter of 20 cm to 35 cm. The diameter of flat, oval stimulation coil 250 is determined by major axis 251 as indicated in Fig. 1C. Accordingly, the width of stimulation coil 250, determined by minor axis 252 as indicated in Fig. 1C, is smaller than 35 cm. As such, stimulation coil 250 is sized and shaped to correspond to at least one foot.

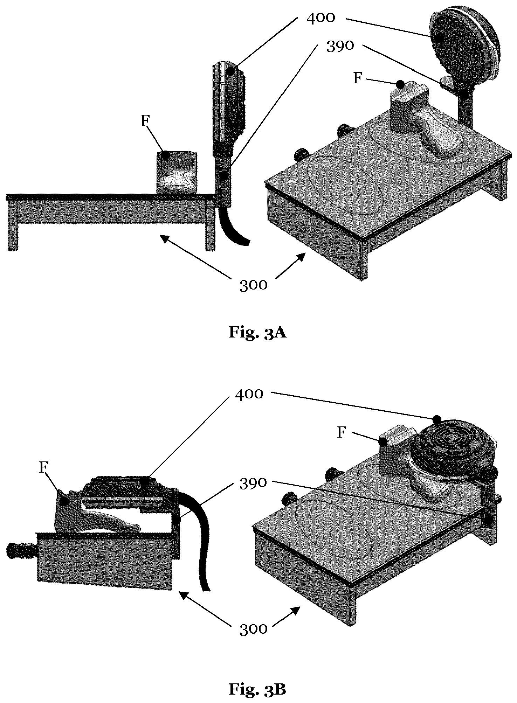

Figs. 3A-D show examples of uses of a magnetic-field applicator 300 according to the present invention in combination with a hand-held applicator 400. Magnetic-field applicator 300 may be identical in construction to magnetic-field applicator 100, such that the above explanations apply *mutatis mutandis.* Hand-held applicator 400 may generally be a common hand-held applicator known from the prior art. Any deviations and/or modifications to hand-held applicator 400, as far as applicable, will be discussed in more detail below. Magnetic-field applicator 300 and hand-held applicator 400 may be part of the same magnetic-stimulation system (not shown). For example, magnetic-field applicator 300 and hand-held applicator 400 may be connected to the same pulse generator. In other examples, magnetic-field applicator 300 and hand-held applicator 400 may also be connected to a separate pulse generator each. The above explanations regarding possible parameters (magnetic field intensity, magnetic field change rate, current, pulse duration, etc.) apply *mutatis mutandis.*

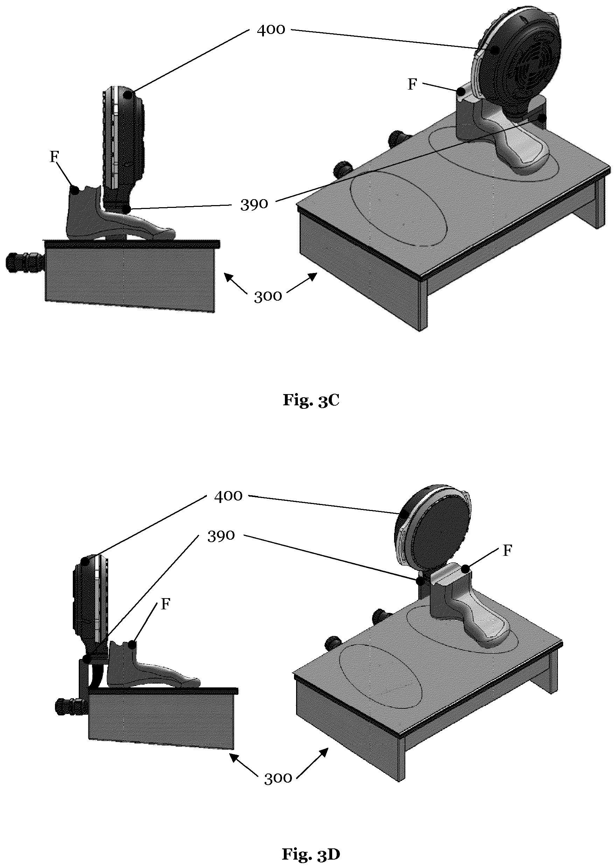

In the example of Fig. 3A, hand-held applicator 400 is used to apply one or more additional pulses to a leg of the individual, below a knee of the leg, specifically to an ankle of a foot F (shown schematically throughout Figs. 3A-D) of an individual. In the example of Fig. 3B, hand-held applicator 400 is used to apply one or more additional pulses to a top of foot F of the individual. In the example of Fig. 3C, hand-held applicator 400 is used to apply one or more additional pulses to the leg of the individual, below the knee of the leg, specifically to a shin (tibia) of the leg (only foot F shown). In the example of Fig. 3D, hand-held applicator 400 is used to apply one or more additional pulses to the leg of the individual, below the knee of the leg, specifically to an Achilles tendon (only foot F shown). Especially in the example of Fig. 3C, magnetic-field applicator 300 and hand-held applicator 400 may provide, in combination, an enhanced urge incontinence treatment. In the examples of Fig. 3A, Fig. 3B and Fig. 3D, magnetic-field applicator 300 and hand-held applicator 400 may provide, in combination, an enhanced treatment of injuries or inflammations in the ankle or the foot of the individual, respectively.

In the examples of Figs. 3A-D, hand-held applicator 400 is held in place using an arm 390 attached to magnetic-field applicator 300. Generally, arm 390 may be attached to magnetic-field applicator 300 by any suitable means. For example, arm 390 may comprise a clamp that receives at least a portion of the treatment surface of magnetic-field applicator 300. Likewise, arm 390 may be integral with magnetic-field applicator 300.

In other examples, hand-held applicator 400 may be held in place manually or by a stand (e.g., a tripod). Additionally or alternatively, hand-held applicator 400 may be strapped to the respective body part of the individual, e.g., the ankle, foot or leg.

In the examples of Figs. 3A-D, hand-held applicator 400 is slightly modified in that its handle has been detached. Instead of being attached to the handle, the body of hand-held applicator 400 is (immediately) attached to arm 390. Generally, hand-held applicator 400, or its body, respectively, may be attached to arm 390 by any suitable means. For example, hand-held applicator 400 may comprise a thread on/at its body that allows to attach both a handle or an arm like arm 390. Likewise, such a thread may be used to attach a stand to hand-held applicator 400 or its body, respectively.

In other examples, an arm like arm 390 or a stand may be adapted to receive (and be attached to) hand-held applicator 400 with a handle attached. For example, an arm like arm 390 or a stand may comprise a clamp which may receive at least a portion of a handle of hand-held applicator 400.

In yet other examples, instead of hand-held applicator 400, there may be one or more other (types of) applicators integrated into, or at least be adapted to be attached to, magnetic-field applicator 300, which one or more other (types of) applicators apply one or more additional pulses to a desired body part of the individual, e.g., a pelvic or pelvic floor region of the individual and/or to a leg of the individual, preferably below a knee of the leg.

## Claims

1. Magnetic-field applicator (100, 300), comprising:
a treatment surface (110) adapted to receive at least one foot in an upright orientation; and
at least one stimulation coil (150a, 150b, 250).

2. Magnetic-field applicator (100, 300) according to claim 1, wherein the at least one stimulation coil (150a, 150b, 250) is positioned below the treatment surface (110).

3. Magnetic-field applicator (100, 300) according to claim 1 or 2, wherein the applicator (100, 300) is adapted to be placed on the ground with the treatment surface (110) pointing substantially upwards, and preferably such that the at least one stimulation coil (150a, 150b, 250) is held at an elevated position with respect to the ground.

4. Magnetic-field applicator (100, 300) according to claim 3, wherein, when the applicator (100, 300) is placed on the ground, at least a portion of the treatment surface (110) and/or at least a portion of the at least one stimulation coil (150a, 150b, 250) encloses a first angle with respect to the ground, preferably of at most 45°, more preferably of at most 15°, most preferably of 2° to 8°, wherein the first angle is optionally adjustable.

5. Magnetic-field applicator (100, 300) according to any of the preceding claims, wherein the treatment surface (110) and/or the at least one stimulation coil (150a, 150b, 250) are movable relative to each other, preferably by one or more motors.

6. Magnetic-field applicator (100, 300) according to any of the preceding claims, wherein a magnetic core is placed inside and/or around the at least one stimulation coil (150a, 150b, 250), wherein the magnetic core is optionally movable relative to the treatment surface (110) and/or the at least one stimulation coil (150a, 150b, 250), preferably by one or more motors.

7. Magnetic-field applicator (100, 300) according to any of the preceding claims, wherein the at least one stimulation coil (150a, 150b, 250) is sized and/or shaped to correspond to the at least one foot.

8. Magnetic-field applicator (100, 300) according to any of the preceding claims, wherein the at least one stimulation coil (150a, 150b, 250) comprises a round shape, a figure-of-eight shape or an oval shape.

9. Magnetic-field applicator (100, 300) according to any of the preceding claims, wherein the treatment surface (110) is adapted to receive two feet in an upright orientation.

10. Magnetic-field applicator (100, 300) according to any of the preceding claims, wherein the at least one stimulation coil (150a, 150b, 250) comprises a first stimulation coil (150a) and a second stimulation coil (150b) placed side-by-side with respect to the treatment surface (110).

11. Magnetic-field applicator (100, 300) according to claim 10, wherein the first stimulation coil (150a) comprises a first orientation and the second stimulation coil (150b) comprises a second orientation, wherein the first stimulation coil (150a) and the second stimulation coil (150b) are arranged such that the first orientation and the second orientation enclose a second angle of 5° to 40°, most preferably of 25° to 35°, wherein the second angle is optionally adjustable.

12. Magnetic-field applicator (100, 300) according to any of the preceding claims, wherein the treatment surface (110) comprises:
at least one bulge adapted to receive at least a part of the at least one foot, preferably a medial part of a forefoot region of the at least one foot, wherein optionally a shape of the at least one bulge is adjustable, and/or
a shape that forms a footbed for the at least one foot, and/or
a biocompatible material.

13. Magnetic-field applicator (100, 300) according to any of the preceding claims, wherein the at least one stimulation coil (150a, 150b, 250) is air cooled and/or liquid cooled.

14. Magnetic-field applicator (100, 300) according to any of the preceding claims, wherein the at least one stimulation coil (150a, 150b, 250) is adapted to generate a magnetic field at the treatment surface (110):
with an intensity of 0.1 to 5 T, preferably with an intensity of 0.5 to 3 T, and/or
with a change rate of 500 to 500,000 T/s, preferably with a change rate of 10,000 to 200,000 T/s, and/or
upon receiving a current of 500 to 10,000 A, preferably upon receiving a current of 3,000 A to 5,000 A, and/or
for a duration of 10 to 900 µs, preferably for a duration of 100 to 500 µs, and/or
wherein the at least one stimulation coil (150a, 150b, 250) comprises a diameter of 15 cm to 40 cm, preferably of 20 cm to 35 cm, and/or
wherein the at least one simulation coil (150a, 150b, 250) comprises an inductance of 0.1 to 10,000 µH, preferably of 1 to 500 µH.

15. Magnetic-stimulation system, comprising:
at least one pulse generator; and
a magnetic-field applicator (100, 300) according to any of the preceding claims, wherein the applicator (100, 300) and/or the at least one stimulation coil (150a, 150b, 250) is connectable to the at least one pulse generator.

16. Method for magnetic stimulation of at least one foot of an individual, comprising:
placing, in an upright orientation, the at least one foot on a treatment surface (110) of a magnetic-field applicator (100, 300) according to any of the claims 1 through 14; and
applying one or more pulses by the applicator (100, 300).

17. Method according to claim 16, wherein the one or more pulses are applied by the applicator while the individual is sitting, preferably on a chair.

18. Method according to claim 16 or 17, further comprising:
applying one or more additional pulses, by one or more additional applicators (400), to a pelvic or pelvic floor region of the individual and/or to a leg of the individual, preferably below a knee of the leg.

19. Use of a magnetic-field applicator (100, 300) according to any of the claims 1 through 14 in an urge incontinence treatment.
